# EUROPEAN PATENT SPECIFICATION

(11) **EP 2 981 831 B1**
(45) Date of publication and mention of the grant of the patent: **21.11.2018**
(21) Application number: 13789356.6
(22) Date of filing: 13.11.2013
(51) Int. Cl.: G01N 33/92

(54) **A METHOD FOR EVALUATING THE LEVEL OF NEUTRALIZATION OF THE BIOLOGICAL ACTIVITY OF LIPOPOLYSACCHARIDES (LPS) IN A SAMPLE**
VERFAHREN ZUR BEURTEILUNG DES NEUTRALISIERUNGSNIVEAUS DER BIOLOGISCHEN AKTIVITÄT VON LIPOPOLYSACCHARIDEN (LPS) IN EINER PROBE
PROCÉDÉ PERMETTANT D'ÉVALUER LE NIVEAU DE NEUTRALISATION DE L'ACTIVITÉ BIOLOGIQUE DES LIPOPOLYSACCHARIDES (LPS) DANS UN ÉCHANTILLON

(30) Priority: 04.04.2013 EP 13305435
(43) Date of publication of application: 10.02.2016
(73) Proprietor: Université de Bourgogne, 21000 Dijon (FR); INSERM - Institut National de la Santé et de la Recherche Médicale, 75013 Paris (FR)
(72) Inventor: LAGROST, Laurent, F-21079 Dijon cedex (FR); PAIS DE BARROS, Jean-Paul, F-21079 Dijon cedex (FR); GAUTIER, Thomas, F-21079 Dijon cedex (FR)
(74) Representative: Cabinet Plasseraud
(86) International application number: PCT/EP2013/073689
(87) International publication number: WO 2014/161613

(56) References cited:
- WO-A1-90/13534
- US-A1- 2012 197 535
- DOROTA POMORSKA ET AL: "ORIGINAL ARTICLES AAEM LEVELS OF BACTERIAL ENDOTOXIN IN AIR OF ANIMAL HOUSES DETERMINED WITH THE USE OF GAS CHROMATOGRAPHY - MASS SPECTROMETRY AND LIMULUS TEST", ANN AGRIC ENVIRON MED, 1 January 2007 (2007-01-01), XP055098546,
- NORBERT BINDING ET AL: "Quantification of bacterial lipopolysaccharides (endotoxin) by GC-MS determination of 3-hydroxy fatty acids", JOURNAL OF ENVIRONMENTAL MONITORING, vol. 6, no. 1, 1 January 2004 (2004-01-01) , pages 65-70, XP055098549, ISSN: 1464-0325, DOI: 10.1039/b309237b
- LI Y ET AL: "Determination of lipid profile in meningococcal polysaccharide using reversed-phase liquid chromatography", JOURNAL OF CHROMATOGRAPHY B: BIOMEDICAL SCIENCES & APPLICATIONS, ELSEVIER, AMSTERDAM, NL, vol. 804, no. 2, 25 May 2004 (2004-05-25), pages 353-358, XP004501593, ISSN: 1570-0232, DOI: 10.1016/J.JCHROMB.2004.01.042
- LAURE TRIBALAT ET AL: "Advantages of LC-MS-MS compared to LC-MS for the determination of nitrofuran residues in honey", ANALYTICAL AND BIOANALYTICAL CHEMISTRY, SPRINGER, BERLIN, DE, vol. 386, no. 7-8, 8 November 2006 (2006-11-08), pages 2161-2168, XP019458146, ISSN: 1618-2650, DOI: 10.1007/S00216-006-0878-3
- ANNA LAWNICZEK WALCZYK ET AL: "Endotoxins and [beta]-glucans as markers of microbiological contamination--characteristics, detection, and environmental exposure.", AAEM. ANNALS OF AGRICULTURAL AND ENVIRONMENTAL MEDICINE, vol. 17, no. 2, 1 December 2010 (2010-12-01), pages 193-208, XP055071031, ISSN: 1232-1966
- NICOLE ZEHETHOFER ET AL: "Plasma free fatty acid profiling in a fish oil human intervention study using ultra-performance liquid chromatography/electrospray ionization tandem mass spectrometry", RAPID COMMUNICATIONS IN MASS SPECTROMETRY, vol. 22, no. 13, 15 July 2008 (2008-07-15) , pages 2125-2133, XP055071030, ISSN: 0951-4198, DOI: 10.1002/rcm.3597
- B. SZPONAR ET AL: "Distribution of 3-Hydroxy Fatty Acids in Tissues after Intraperitoneal Injection of Endotoxin", CLINICAL CHEMISTRY, vol. 49, no. 7, 1 July 2003 (2003-07-01), pages 1149-1153, XP055071032, ISSN: 0009-9147, DOI: 10.1373/49.7.1149
- A Sonesson ET AL: "Comparison of the limulus amebocyte lysate test and gas chromatography-mass spectrometry for measuring lipopolysaccharides (endotoxins) in airborne dust from poultry-processing industries", Applied and Environmental Microbiology, 1 May 1990 (1990-05-01), pages 1271-1278, XP055336800, UNITED STATES Retrieved from the Internet: URL:http://aem.asm.org/content/56/5/1271.f ull.pdf#page=1&view=FitH [retrieved on 2017-01-19]
- Opal Sm: "The host response to endotoxin, antilipopolysaccharide strategies, and the management of severe sepsis. - PubMed - NCBI", International journal of medical microbiology, 1 September 2007 (2007-09-01), pages 1-1, XP055337038, Retrieved from the Internet: URL:https://www.ncbi.nlm.nih.gov/pubmed?te rm="International+journal+of+medical+micro biology+:+IJMM"[Jour]+AND+297[volume]+AND+ 365[page]&cmd=detailssearch [retrieved on 2017-01-19]
- JEAN-PAUL PAIS DE BARROS ET AL: "Quantitative lipopolysaccharide analysis using HPLC/MS/MS and its combination with the limulus amebocyte lysate assay", JOURNAL OF LIPID RESEARCH, vol. 56, no. 7, 1 July 2015 (2015-07-01), pages 1363-1369, XP055423083, US ISSN: 0022-2275, DOI: 10.1194/jlr.D059725

## Description

### FIELD OF THE INVENTION

The present invention relates to LPS quantification.

### BACKGROUND OF THE INVENTION

LipoPolySaccharide (LPS) quantitation is a key point in any endotoxemia survey. *Limulus Amebocyte Lysate* (LAL) assay or alternatively Gas Chromatography Mass Spectrometry (GCMS) were previously used to quantitate LPS in different media. However, these methods have led to mixed results and do not bring a clear and comprehensive picture on their own. On the one hand, the LAL assay relies on the biological activity of LPS and is dependent both on total amounts of LPS and on the ability of endogenous factors to bind and/or neutralize LPS. On the other hand, the GCMS assays allows a direct quantitation of LPS concentration but has major drawbacks (including potentially explosive methanolysis and a derivatization step), requires further upgrading, and most importantly does not allow assessment of LPS neutralization in biological media.

LPS are external wall components of Gram-negative bacteria. They are responsible for the host immune response involving the production of proinflammatory cytokines by macrophages and supplementary leucocyte recruitment. In the case of uncontrolled inflammatory response, these events have harmful consequences and can lead to endotoxic shock. Endotoxemia surveys are limited by the lack of practicable, reliable and performing LPS quantitation methods in body fluids and tissues. Today, the LAL assay and the GCMS method with methanolysis are available for LPS quantitation. The LAL assay is not a LPS mass assay per se but rather behaves as a LPS biological activity assay. It has a number of limitations in terms of accuracy and reproducibility. The GCMS assay is time-consuming, expensive and involves toxic reagents. In addition, its sensitivity, linearity and threshold limit are likely improvable (see refs 1-11).

For LPS quantitation by GCMS, 3-hydroxymyristic acid or 3-hydroxytetradecanoic acid is used as a specific component of LPS. Indeed, the LPS molecule is constituted of an oligosaccharidic chain (with a composition and length which depend on bacterial strains) and of a lipid A moiety (which accounts for its proinflammatory potential). According to the R. Chaby's classification, among the LPS molecules from 66 bacteria species listed, 39 contain up to four molecules of 3-hydroxymyristic acid (as it is the case for O55:B5 E. Coli) (see ref. 12)

### SUMMARY OF THE INVENTION

The present invention relates to a method for evaluating the level of neutralization of the biological activity of lipopolysaccharides (LPS) in a sample, wherein the total LPS concentration is measured by mass spectrometry (such as GCMS, LCMS, preferably LCMS/MS) and LPS biological activity is measured by a biological assay (such as *Limulus Amebocyte Lysate* (LAL) assay), and wherein the level of neutralization is determined by comparing the concentration results obtained by mass spectrometry and the activity results obtained with the LPS biological activity assay.

### DETAILED DESCRIPTION OF THE INVENTION

The sample can be any sample which might contain LPS, for example a water sample, an air dust sample, a food sample (e.g., food matrices, wine, dairy products), or any biological sample (e.g., biological fluids, such as a blood sample (e.g., plasma or whole blood sample), tissues...), drugs, soils, or packaging. Typically the sample is a biological sample, such as a blood sample (e.g., a plasma or a whole blood sample).

In a preferred embodiment the LPS concentration in the sample is determined by quantifying by high-performance liquid chromatography coupled to tandem mass spectrometry (LCMS/MS or LCMS²) the 3-hydroxytetradecanoic acid released after hydrolysis of the LPS.

Typically, the 3-hydroxytetradecanoic acid is released after acid hydrolysis of the LPS.

Typically after the hydrolysis of the LPS, the resulting 3-hydroxytetradecanoic acid is extracted with an organic solvent.

For the quantification of 3-hydroxytetradecanoic acid by LCMS/MS, any conditions which allow the separation of fatty acids by high-performance liquid chromatography and which are compatible with MS/MS detection may be used. Typically the separation is performed by reversed phase HPLC. For example, the skilled person may use a column filled with modified silica to make it non-polar by attaching long hydrocarbon chains to its surface - typically with either 8 or 18 carbon atoms in them. For the elution a gradient of eluents may be used, e.g. a gradient made with an acetate ammonium buffer and acetonitrile.

For the MS/MS detection, any mass spectrometer which is able to detect 3-hydroxytetradecanoic acid may be used. Triple quadrupole mass spectrometers may for example be used.

The invention relates to a method for evaluating the level of neutralization of the biological activity of lipopolysaccharides (LPS) in a sample, wherein the LPS are quantified by mass spectrometry and with a LPS biological activity assay, and wherein the level of neutralization is determined by comparing the results obtained by mass spectrometry and with the results obtained with the LPS biological activity assay.

Typically the mass spectrometry is Gas Chromatography Mass Spectrometry (GCMS) or Liquid Chromatography Mass Spectrometry (LCMS).

In a preferred embodiment, the mass spectrometry is LCMS/MS and the LPS are quantified by the LCMS/MS method described above.

LPS biological activity assays are well known, these include, for example, the rabbit pyrogen test, the fever index, animal lethality, epinephrine skin test, synovial inflammatory response, the LAL assay and LAL like assays such as the PyroGene™ assay of Lonza, Fluorescence assays (EndoLISA, Pyrogene, EndoZyme), enzymatic tests (for example HEK-Blue LPS detection kit).
In a preferred embodiment the LPS biological activity assay is the LAL assay. Limulus amebocyte lysate (LAL) is an aqueous extract of blood cells (amebocytes) from the horseshoe crab, *Limulus polyphemus.* LAL reacts with LPS. This reaction is the basis of the LAL assay.
The expression "*Limulus Amebocyte Lysate* (LAL) assay" should be understood broadly, it encompasses any assay which makes use of LAL or of an amebocyte lysate from of horseshoe crabs of another species such as *Tachypleus gigas* to detect LPS or use of recombinant proteins, such as recombinant Factor C (rFC), involved in the LAL clotting cascade.
*Limulus Amebocyte Lysate* (LAL) assays are a well-known in the art (see for example LAL Update October 2005 • Volume 22, No.3 or International Food Research Journal 19(2): 423-425 (2012)).

The combined method for evaluating the level of endotoxins and the lipopolysaccharide (LPS) neutralizing capacity of a sample may be used for the analysis of biological fluids, tissues, food matrices, wine, dairy products, ambient air, water, drugs, soils, packaging ....

The combined method may be used in the fields of medicine, fundamental and applied research, oenology, pharmaceutical industry, foodstuff and beverage industry (including dairy industry), control and treatment of water (including drinking, industrial and waste water), maintenance of pipe water systems, ...
As examples, the method may be used to improve diagnosis and patient care during:
- Preoperative workup (for instance prior to a surgical operation, for instance including bowel surgery, lung surgery, cardiac surgery with or without cardiopulmonary bypass, laparotomy, elective surgery, aortic aneurysm repair, or liver resection);
- Post-perfusion syndrome after cardiopulmonary bypass;
- Hemodialysis (with respiratory and circulatory changes);
- Graft-vs-host disease (GvHD) (in particular after bone marrow transplant);
- Sepsis or septic shock;
- Systemic Inflammatory Response Syndrome (SIRS);
- Apheresis (for instance monitoring of blood samples); or
- any other pathophysiological situation which is or can be associated with endotoxemia (including surgery, infectious diseases, dyslipidemia, metabolic disorders, obesity, metabolic syndrome, inflammatory bowel disease, alcoholic hepatitis,...)

The method can be used for example:
- To evaluate the endotoxin-scavenging potential of any biological sample;
- To evaluate the propensity of individuals to fight infection;
- To evaluate any treatment which is supposed to neutralize and/or eliminate endotoxins;
- To evaluate simultaneously the endotoxin content as well as the endotoxin-neutralizing or endotoxin-scavenging property of any sample (including water, soil, any biological sample, food, beverages,...);
- To assess the endotoxin-neutralizing or endotoxin-scavenging property of any material (for instance sorbents, containers, packaging,...);
- To measure the bacterium-binding efficacy of any material; or
- To check fermentation processes in dairy foods and wine.

The method has several advantages over previous ones, mostly based on biological activity measurements. The present method allows:
- the identification of false negative and false positive results that may appear through single activity measurements;
- the monitoring of the endotoxin-binding capacity of media;
- the simultaneous determination of LPS total concentration, LPS biological activity, and LPS-neutralizing capacity; and
- the diagnosis, follow-up and therapeutic management of patients (for instance during sepsis, septic shock, extra-renal purification).

In the following, the invention will be illustrated by means of the following examples and figures.

### FIGURE LEGENDS

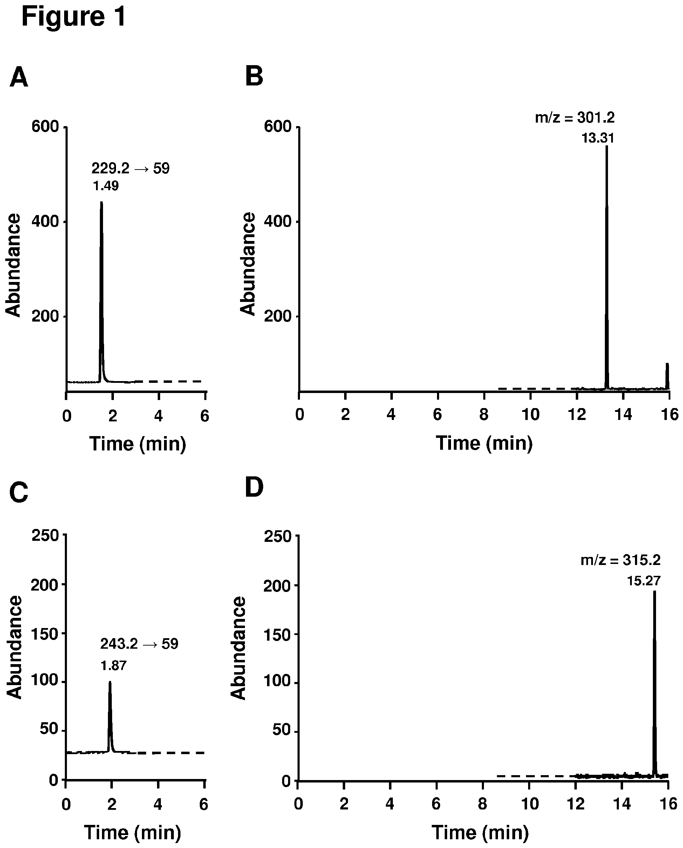

**Figure 1****. Representative chomatograms of hydroxytetradecanoic acid and hydroxytridecanoic acid obtained by LCMS² and GCMS analysis.** LCMS² (A and C) and GCMS (B and D) were used to generate chromatograms with 50ng of 3-hydroxytridecanoic acid used as internal standard (A and B) and with 3-hydroxytetradecanoic acid extracted from 25µl of plasma from mice that were injected intraperitoneally with LPS (B and D). Hydrolysis, fatty acid extraction, derivatization, LCMS² MRM and GCMS SIM analysis conditions are described under material and methods. Panel A: extracted chromatogram corresponding to the 229.2 → 59 transition of 3OH-tridecanoic acid (RT = 1.49 min) obtained by LCMS². Panel B: extracted chromatogram corresponding to the [M-15]⁺ ion (m/z = 301.2) of 3OTMS-tridecanoic-methyl ester (RT = 13.31 min) obtained by GCMS. Panel C: extracted chromatogram corresponding to the 243.2 → 59 transition of 3-hydroxy-tetradecanoic acid (RT = 1.87 min) obtained by LCMS². Panel D: extracted chromatogram corresponding to the [M-15]⁺ ion (m/z = 315.2) of 3OTMS-tetradecanoic-methyl ester (RT = 15.27 min) obtained by GCMS.

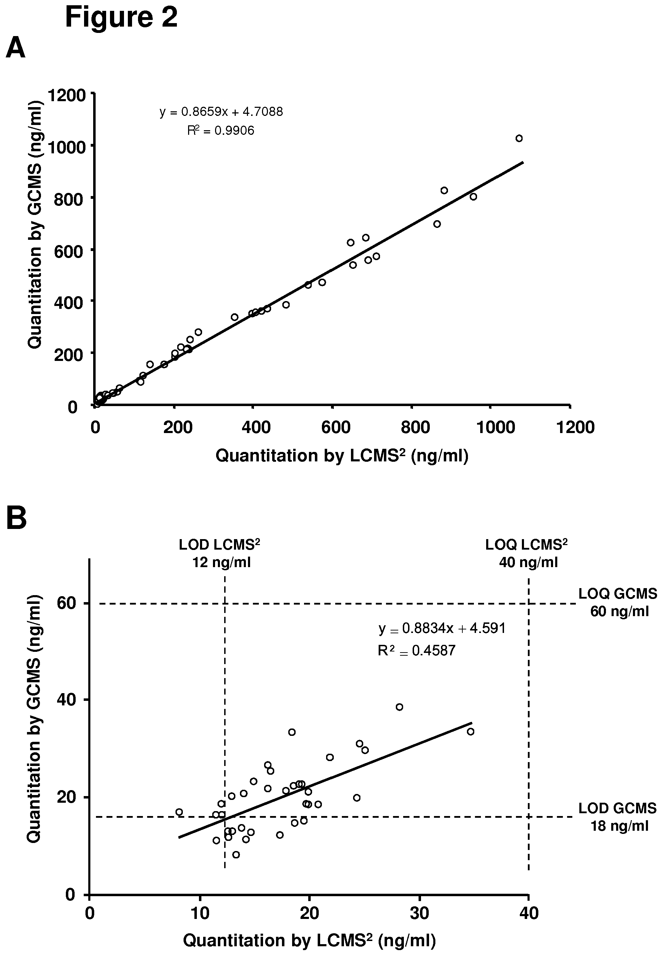

**Figure 2****. Comparison of sensitivity and accuracy of LCMS² and GCMS techniques for LPS quantitation in plasma samples.** Mouse plasma samples containing variable amounts of LPS were obtained after intraperitoneal injection of LPS from *Escherichia coli* (O55:B5) and were submitted to HCl hydrolysis and lipid extraction as described under materials and methods. One aliquot of the resulting preparation was analyzed by LCMS² and another aliquot was analyzed by GCMS for quantitation of 3-hydroxymyristic acid (see materials and methods). Panel A shows the linear regression curve built by plotting LPS values determined by GCMS against LPS values determined by LCMS². Panel B shows the linear regression curve as well as the determination of the limit of detection (LOD) and of the limit of quantification (LOQ) of LCMS² and GCMS by extracting the lowest values (0-40 ng/ml range) of LPS levels in mouse plasmas shown in panel A.

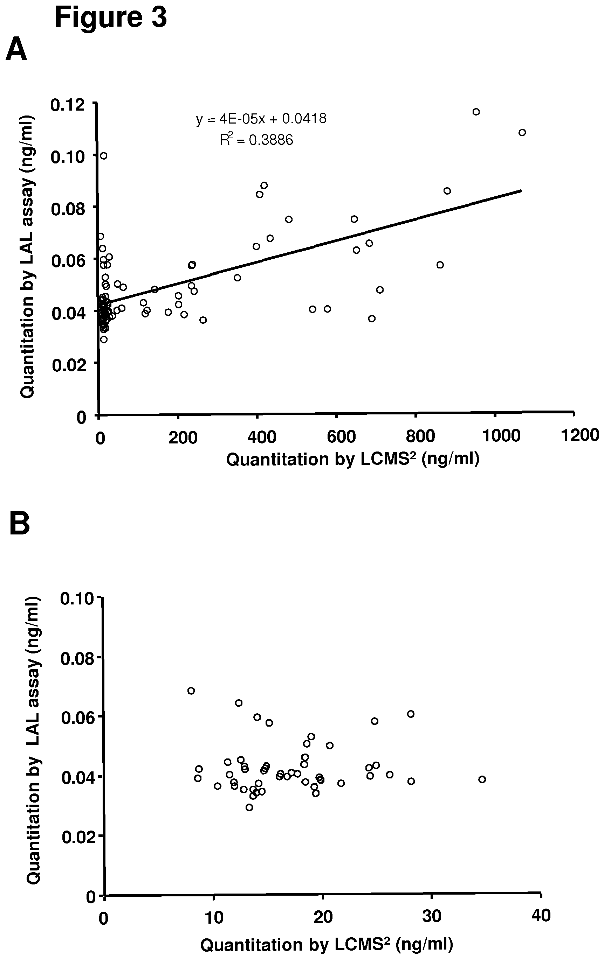

**Figure 3****. Comparison of sensitivity and accuracy of LCMS² and LAL techniques for LPS quantitation in plasma samples.** Mouse plasma samples containing variable amounts of LPS were obtained after intraperitoneal injection of LPS from *Escherichia coli* (O55:B5) and blood collection by retroorbital puncture. For each sample, one aliquot was directly analyzed by LAL assay and another aliquot was submitted to HCl hydrolysis and lipid extraction prior to quantitation of 3-hydroxymyrisitc acid by LCMS² (see materials and methods). Panel A shows the linear regression curve built by plotting LPS values determined by LAL assay against LPS values determined by LCMS². Panel B shows the lowest values (0-40 ng/ml range) of LPS levels in mouse plasmas extracted from panel A; no significant correlation between LAL and LCMS² could be observed in the low range of LPS levels.

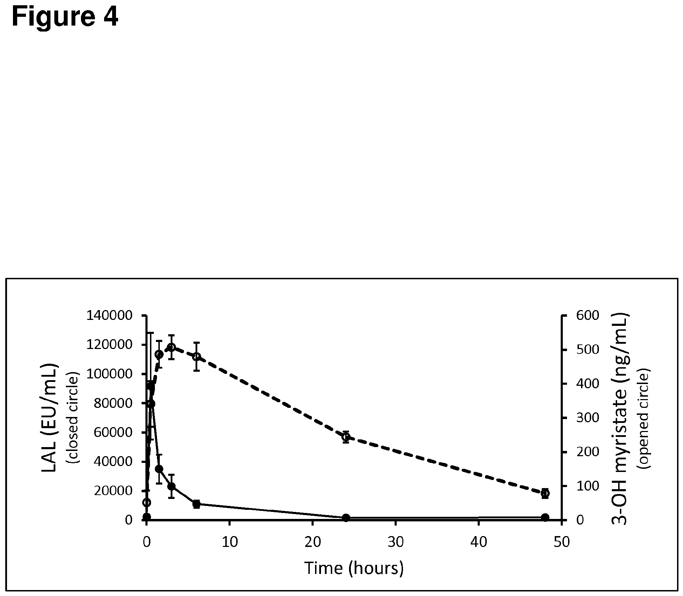

**Figure 4****. Plasma kinetic curves of LPS levels determined by LCMS² and LAL assay in wild-type mice.**
   Wild-type C57BL/6J mice (n=6) were injected intraperitoneally with LPS from *Escherichia coli* (O55:B5, 1 mg/kg body weight). Blood samples were drawn by retroorbital puncture before injection (time 0) and 0.5, 1, 3, 6, 24 and 48 hours after injection and plasmas were obtained after centrifugation. One aliquot was analyzed by LAL assay (closed circles) and another one by LCMS² (opened circles) for LPS quantitation as described in materials and methods. Values are expressed in ng/ml (for LCMS²) or in EU/ml (for LAL) and are the means of 6 animals ± SEM.

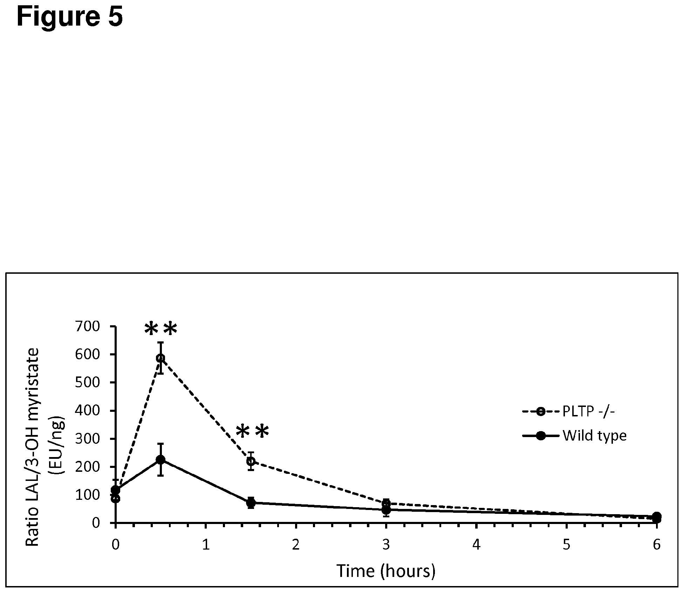

**Figure 5****. Ratio of LPS activity to LPS mass concentration determined in wild-type and PLTP-deficient mice.**
   Blood samples were drawn by retroorbital puncture before injection (time 0) and 0.5, 1.5, 3, and 6 hours after injection of *Escherichia coli* LPS (O55:B5, 1 mg/kg body weight). Plasmas were obtained after centrifugation. LPS biological activity (LAL assay) to LPS mass concentration (direct 3-OH-myristate assay) ratios were determined over the 6-hour period in wild-type (full line) and PLTP-deficient (PLTP-/-) mice (dotted line). Vertical bars are mean ± sem of 6 animals. **P<0.01 significantly different from WT mice.

### EXAMPLES

### Experimental procedures

### LPS

The dry extract of LPS (*Escherichia coli* serotype O55:B5; Sigma Chemicals) was suspended in endotoxin-free, 0.15 M sodium chloride and vigorously mixed for 15 min before use.

### Animals

Wild-type, C57BL/6J mice (3 month-old) were generated by Charles River. Animals were fed with a standard chow diet (A03 diet; Safe, Augy, France). Animals had free access to water and food. All experiments involving animals were performed in accordance with institutional guidelines and approved by the University of Burgundy's Ethics Committee on the Use of Laboratory Animals (protocol number 2511).

### LPS injection and blood sampling

All materials and glasswares were apyrogen or heat-depyrogenated before use (at least 30 min at 250°C in a hot air oven), and all of the reagents used were of 'endotoxin-free" grade. The LPS was injected intraperitoneally into mice (single doses). Blood was then collected at the indicated times by retroorbital puncture. Plasma was obtained by blood centrifugation (10 min, 2000 g at 4°C)

### LPS quantification

LPS in plasma was assayed by using either a biological activity assay (*Limulus Amebocyte Lystae* (LAL) kit, QCL-1000; Lonza, Walkersville, MD USA) or the direct quantitation of 3-hydroxymyristic acid by GCMS or by LCMS/MS (with hydrochloric acid hydrolysis in both cases).

### Gas Chromatography-Mass Spectrometry with hydrochloric acid hydrolysis

LPS concentration was determined by direct quantitation of 3-O-trimethylsilyl ether-tetradecanoic methyl ester by GC-MS.
Briefly, 25 µl of plasma spiked with 50 ng of internal standard (3-hydroxytridecanoic acid 10 µg/ml in ethanol) was hydrolysed with 75 µl of NaCl 150 mM and 300 µl of HCl 8M for 4 hours at 90°C. Free fatty acids were then extracted with 600 µl of distilled water and 5 ml of hexane. After evaporation of the hexanic phase, fatty acids were methylated with 250 µl of BF₃/MeOH 1/9 for 30 min at 60°C. Methylation solution was then evaporated under N₂ stream. Dried extract was further incubated for 1 hour at 80°C with 100 µl of N,O-Bis(trimethylSilyl)TriFluoroAcetamide/TriMethyl-ChloroSilane (BSTFA/TMCS, 4:1) solution for derivatization. Silylating solution was then evaporated under N₂ stream. Finally 100 µl of hexane was added and 1 µl of the mixture was injected in split/splitless mode (split ratio 10/1) as previously described (Gautier T et al. JBC, 2008, 27, 18702-10) on an Agilent GC 6890, MSD 5973 device.

### High Performance Liquid Chromatography-tandem Mass Spectrometry

LPS concentration was determined by direct quantitation of 3-hydroxytetradecanoic acid by LCMS².
Briefly, 25 µl of plasma spiked with 50 ng of internal standard (3-hydroxytridecanoic acid 10 µg/ml in ethanol) was hydrolysed with 75µl of NaCl 150 mM and 300 µl of HCl 8M for 4 hours at 90°C. Free fatty acids were then extracted with 600 µl of distilled water and 5 ml of hexane. After vacuum evaporation of the hexanic phase, fatty acids were dissolved in 100 µl of a 40%A/60%B eluent mixture (eluent A: acetate ammonium 5 mM pH5.0 ; eluent B : acetonitrile / acetate ammonium 5 mM pH7.3 96.7%/3.3% (Zehethofer N. et al. Rapid Commun Mass Spectrom. 2008;22:2125-33).
Fatty acid separation was performed in an Infinity 1200 HPLC binary system (Agilent) equipped with a Poroshell 120 EC C18 100 x 4.6 mm 1.7 µm column (Agilent) set at 30°C. The sample volume injected was 10 µl. A 7 min eluent gradient was performed as follows: from 0 to 0.5 minutes the flow was maintained constant at 1 ml/min of 80% B ; then the proportion of B increased linearly up to 100% in 1 min, concomitantly flow rate was decreased to 0.5 ml/min ; these conditions were maintained constant for 1 min ; then flow rate was increased up to 1 ml/min for 2.5 more minutes; finally column was re-equilibrated with 80%B at 1ml/min for 2.5 minutes.
MS/MS detection was performed using a QQQ 6460 triple quadrupole mass spectrometer (Agilent) equipped with a JetStream ESI source in negative mode (Gas temperature 300°C, Gaz flow 10 L/min, Nebulizer 20 psi, Sheath gas temperature 200°C, Sheath gas flow 11 L/min, Capillary 3500 V). Nitrogen was used as collision gas.
Mass spectrometer was setup in MRM mode for the quantification of selected ions as follows:
3-hydroxytetradecanoic acid: precursor ion 243.2 Da; product ion 59 Da, fragmentor 93 V, collision cell 9 Ev.
3-hydroxytridecanoic acid: precursor ion 229.2 Da; product ion 59 Da, fragmentor 110 V, collision cell 10 eV.

### LAL assay

Biological activity of LPS was quantified by an endpoint chromogenic LAL test (Lonza, QCL-1000, 50-648U). The reagents were rehydrated and equilibrated as recommended by the manufacturer.
50 µl of plasma diluted 1:10,000 with endotoxin-free grade water were dispensed in each well of a 96-well plate. At initial time (t=0min), 50 µl of LAL reagent were added in each well. The plate was shaked and incubated at 37°C. After 10 minutes (t=10min), 100 µl of 37°C-warmed chromogenic substrate were put in each well. After 6 minutes (t=16min), 100 µl of stop reagent (acetic acid, 25% v/v glacial acetic acid in water) were dispensed in each well. The plate was shaked. Aborbance was measured at 405 nm on a spectrophotomer (Perkin Elmer, Victor 3).

### Results

LCMS² is clearly superior to GCMS for measuring LPS levels. The limits of detection (LOD) and of quantification (LOQ) of LCMS² are much lower than GCMS. Furthermore, LCMS² is less time-consuming than GCMS and does not involve toxic reagents.
Plasma LPS levels as measured by either LCMS² or LAL assays increased rapidly following the single, intraperitoneal injection in wild-type mice. Whereas LPS level peaked a few hours after injection with either assay, clear differences appeared over longer times. Indeed, biological activity of LPS as measured by the LAL assay came back to barely detectable levels 6 hours after injection. In contrast, and unlike LAL data, direct quantitation of 3-hydroxymyristate by LCMS² revealed that LPS were actually maintained at elevated levels much longer in the plasma compartment of wild-type mice, in this case with substantial/high levels of LPS being still detectable at the 24-hr time point. Most importantly, quantitative analysis by LCMS² revealed that maximal plasma levels of LPS were measured after 6 hours, thus indicating that 1) the bulk of circulating LPS was still present in the plasma compartment after 6 hours, and 2) that the LAL assay produced false negative results at the same time-point. Overall, the combination of the LAL and LCMS² analyses comes in support of an intrinsic capacity of plasma to neutralize the activity of LPS which become undetectable by the classical LAL assay, but are still detectable by the LCMS² assay. Importantly, the ratio at 0.5-hour and 1.5-hour time points was significantly lower in wild-type mice than in PLTP-deficient mice which are known to display an impaired ability to detoxify LPS. It indicates that the mass to activity LPS ratio is relevant in assessing the neutralization of LPS in vivo. The rapid and efficient disaggregation and neutralization of LPS in plasma occur prior to their transport back to the liver for ultimate detoxification. Impaired LAL activity as observed here is likely to rely on the previously recognized ability of endogenous plasma lipoproteins to bind and neutralize LPS.
In conclusion, combined results of the LAL and LCMS² analyses bring to the fore a novel way to assess the LPS neutralization capacity of biological samples and to figure what the proinflammatory potential of LPS molecules may be when present in various biological media.

### REFERENCES

Throughout this application, various references describe the state of the art to which the invention pertains.
1. Lindsay GK, Roslansky PF, Novitsky TJ. Single-step, chromogenic Limulus amebocyte lysate assay for endotoxin. J Clin Microbiol 1989 ; 27 : 947-951.
2. Maitra KS, Schotz MC, Yoshikawa TT, Guze LB. Determination of lipid A and endotoxin in serum by mass spectroscopy. Proc Natl Acad Sci U S A 1978 ; 75, 7 : 3993-3997.
3. Brandtzaeg P, Bryn K, Kierulf P, Øvstebø R, Namork E, Aase B, et al. Meningococcal endotoxin in lethal septic shock plasma studied by gas chromatography-mass spectrometry, ultracentrifugation and electron microscopy. J Clin Invest 1992 ; 89 : 816-823.
4. Szponar B, Norin E, Midtvedt T, Larsson L. Limitations in the use of 3-hydroxy fatty acid analysis to determine endotoxin in mammalian samples. J Microbiol Methods 2002 ; 50 : 283-289.
5. Saraf A, Larsson L. Use of gas chromatography/ion-trap tandem mass spectrometry for the determination of chemical markers of microorganisms in organic dust. J Mass Spectrom 1996; 31 : 389-396.
7. Saraf A, Park J-H, Milton DK, Larsson L. Use of quadrupole GC-MS and ion trap GC-MS-MS for determining 3-hydroxy fatty acids in settled house dust: relation to endotoxin activity. J Environ Monit 1999 ; 1 : 163-168.
6. Jones PM, Quinn R, Fennessey PV, Tjoa S, Goodman SI, Fiore S, et al. Improved stable isotope dilution-gas chromatography-mass spectrometry method for serum or plasma free 3-hydroxy fatty acids and its utility for the study of disorders of mitochondrial fatty acid β-oxidation. Clin Chem 2000 ; 46 : 149-155.
7. Costa CG, Dorland L, Holwerda U, Tavares de Almeida I, Poll-The B-T, et al. Simultaneous analysis of plasma free fatty acids and their 3-hydroxy analogs in fatty acid β-oxidation disorders. Clin Chem 1998 ; 44 : 463-471.
8. Maitra SK, Nachum R, Pearson FC. Establishment of β-hydroxy fatty acids as chemical marker molecules for bacterial endotoxin by gas chromatography-mass spectrometry. Appl Environ Microbiol 1986 ; 52 : 510-514.
9. Szponar B, Kras'nik L, Hryniewiecki T, Gamian A, Larsson L. Distribution of 3-Hydroxy Fatty Acids in Tissues after Intraperitoneal Injection of Endotoxin. Clin Chem 2003 ; 49 : 7, 1149-1153.
10. Sonesson A., Larsson L., Shütz A., Hagmar L., Hallberg T. Comparison of the Limulus Amebocyte Lysate Test and Gas Chromatography-Mass Spectrometry for Measuring Lipopolysaccharides (Endotoxins) in Airborne Dust from Poultry-Processing Industries. Appl Environ Microbiol 1990 ; 56 : 5, 1271-1278.
11. Reynolds SJ, Milton DK, Heederik D, Thorne PS, Donham KJ, Croteau EA, Kelly KM, Douwes J, Lewis D, Whitmer M, Connaughton I, Koch S, Malmberg P, Larsson BM, Deddens J, Saraf A, Larsson L. Interlaboratory evaluation of endotoxin analyses in agricultural dusts---comparison of LAL assay and mass spectrometry. J Environ Monit 2005 ; 7: 1371-1377.
12. Kirschner D, Que Hee SS, Clark CS. Method for detecting the 3-hydroxymyristic acid component of the endotoxins of Gram-negative bacteria in compost samples. Am Ind Hyg Assoc J 1985 ; 46: 741-746.
13. Chaby R, Girard R. Mechanisms of cell activation by structurally atypical bacterial lipopolysaccharides and glycolipid substitutes. Curr Trends Immunol 2003 ; 5 : 91-108.
14. Zehethofer N, Pinto DM, Volmer DA. Plasma free fatty acid profiling in a fish oil human intervention study using ultra-performance liquid chromatography/electrospray ionization tandem mass spectrometry. Rapid Commun Mass Spectrom. 2008 Jul;22(13):2125-2133.
15. Gautier T, Klein A, Deckert V, Desrumaux C, Ogier N, Sberna AL, Paul C, Le Guern N, Athias A, Montange T, Monier S, Piard F, Jiang XC, Masson D, Lagrost L. Effect of plasma phospholipid transfer protein deficiency on lethal endotoxemia in mice. J Biol Chem. 2008 Jul 4;283(27):18702-10.

## Claims

1. A method for evaluating the level of neutralization of the biological activity of lipopolysaccharides (LPS) in a sample, wherein the LPS are quantified by mass spectrometry and with a LPS biological activity assay, and wherein the level of neutralization is determined by comparing the results obtained by mass spectrometry and with the results obtained with the LPS biological activity assay.

2. The method according claim 1, wherein the LPS biological activity assay is a *Limulus Amebocyte Lysate* (LAL) assay.

3. The method according to claim 1 or 2, wherein the sample is a biological sample from a patient in a pathophysiological situation which is or can be associated with endotoxemia.

4. The method according to claim 3, wherein the patient is a patient with systemic inflammatory response syndrome (SIRS).

5. The method according to any of claims 1-4, wherein the mass spectrometry is Gas Chromatography Mass Spectrometry (GCMS) or Liquid Chromatography Mass Spectrometry.

6. The method according to claim 5, wherein the LPS concentration in the sample is determined by quantifying by high-performance liquid chromatography coupled to tandem mass spectrometry (LCMS/MS or LCMS²) the 3-hydroxytetradecanoic acid released after hydrolysis of the LPS and with a LPS biological activity assay, and wherein the level of neutralization is determined by comparing the results obtained by LCMS/MS with the results obtained with the LPS biological activity assay.

7. The method according to claim 6, wherein the 3-hydroxytetradecanoic acid is released after acid hydrolysis of the LPS.

8. The method according to claim 6 or 7, wherein after the hydrolysis of the LPS, the resulting 3-hydroxytetradecanoic acid is extracted with an organic solvent.

9. The method according to any of claims 6-8, wherein the concentration of the 3-hydroxytetradecanoic acid is determined by LCMS/MS before and after the LPS hydrolysis, the concentration of 3-hydroxytetradecanoic resulting from the LPS hydrolysis being determined by comparing the concentration of 3-hydroxytetradecanoic after and before hydrolysis.

## Patentansprüche

1. Verfahren zur Bewertung des Neutralisationsgrades der biologischen Aktivität von Lipopolysacchariden (LPS) in einer Probe, worin die LPS durch Massenspektrometrie und mit einem LPS-Assay auf biologische AAktivität quantifiziert werden, und worin der Neutralisationsgrad durch Vergleichen der Ergebnisse die durch Massenspektrometrie erhalten wurden, mit den Ergebnissen, die mit dem LPS-Assay auf biologische Aktivität erhalten wurden, bestimmt wird.

2. Verfahren nach Anspruch 1, worin der LPS-Assay auf biologische Aktivität ein *Limulus-Amöbozyten-Lysat* (LAL)-Assay ist.

3. Verfahren nach Anspruch 1 oder 2, worin die Probe eine biologische Probe eines Patienten in einer pathophysiologischen Situation ist, die mit Endotoxämie verbunden ist oder verbunden sein kann.

4. Verfahren nach Anspruch 3, worin der Patient ein Patient mit systemischem inflammatorischem Response-Syndrom (SIRS) ist.

5. Verfahren nach einem der Ansprüche 1-4, worin die Massenspektrometrie Gaschromatographie-Massenspektrometrie (GCMS) oder Flüssigchromatographie-Massenspektrometrie ist.

6. Verfahren nach Anspruch 5, worin die LPS-Konzentration in der Probe bestimmt wird durch Quantifizierung mittels Hochleistungs-Flüssigchromatographie gekoppelt mit Tandem-Massenspektrometrie (LCMS/MS oder LCMS²) der nach Hydrolyse der LPS freigesetzten 3-Hydroxytetradecansäure und mit einem LPS-Assay auf biologische Aktivität, und worin der Neutralisationsgrad bestimmt wird, durch Vergleichen der Ergebnisse, die durch LCMS/MS erhalten wurden mit den Ergebnissen, die mit dem LPS-Assay auf biologische Aktivität erhalten wurden.

7. Verfahren nach Anspruch 6, worin die 3-Hydroxytetradecansäure nach saurer Hydrolyse der LPS freigesetzt wird.

8. Verfahren nach Anspruch 6 oder 7, worin nach der Hydrolyse der LPS die resultierende 3-Hydroxytetradecansäure mit einem organischen Lösungsmittel extrahiert wird.

9. Verfahren nach einem der Ansprüche 6-8, worin die Konzentration der 3-Hydroxytetradecansäure durch LCMS/MS vor und nach der LPS-Hydrolyse bestimmt wird, wobei die Konzentration von 3-Hydroxytetradecansäure aus der LPS-Hydrolyse durch Vergleich der Konzentration von 3-Hydroxytetradecansäure nach und vor der Hydrolyse bestimmt wird.

## Revendications

1. Procédé d'évaluation du niveau de neutralisation de l'activité biologique de lipopolysaccharides (LPS) dans un échantillon, dans lequel les LPS sont quantifiés par spectrométrie de masse et avec un dosage d'activité biologique de LPS, et dans lequel le niveau de neutralisation est déterminé en comparant les résultats obtenus par spectrométrie de masse et les résultats obtenus avec le dosage d'activité biologique de LPS.

2. Procédé selon la revendication 1, dans lequel le dosage d'activité biologique de LPS est un dosage *Limulus Amebocyte Lysate* (LAL).

3. Procédé selon la revendication 1 ou 2, dans lequel l'échantillon est un échantillon biologique provenant d'un patient dans une situation pathophysiologique qui est ou peut être associée à une endotoxémie.

4. Procédé selon la revendication 3, dans lequel le patient est un patient ayant un syndrome de réponse inflammatoire systémique (SIRS).

5. Procédé selon l'une quelconque des revendications 1 à 4, dans lequel la spectrométrie de masse est une chromatographie en phase gazeuse-spectrométrie de masse (GCMS) ou une chromatographie en phase liquide-spectrométrie de masse.

6. Procédé selon la revendication 5, dans lequel la concentration en LPS dans l'échantillon est déterminée par quantification par chromatographie liquide haute performance couplée à une spectrométrie de masse tandem (LCMS/MS ou LCMS²) de l'acide 3-hydroxytétradécanoïque libéré après l'hydrolyse des LPS et avec un dosage d'activité biologique de LPS, et dans lequel le niveau de neutralisation est déterminé en comparant les résultats obtenus par LCMS/MS aux résultats obtenus avec le dosage d'activité biologique de LPS.

7. Procédé selon la revendication 6, dans lequel l'acide 3-hydroxytétradécanoïque est libéré après l'hydrolyse acide des LPS.

8. Procédé selon la revendication 6 ou 7, dans lequel après l'hydrolyse des LPS, l'acide 3-hydroxytétradécanoïque résultant est extrait avec un solvant organique.

9. Procédé selon l'une quelconque des revendications 6 à 8, dans lequel la concentration en acide 3-hydroxytétradécanoïque est déterminée par LCMS/MS avant et après l'hydrolyse des LPS, la concentration en 3-hydroxytétradécanoïque résultant de l'hydrolyse des LPS étant déterminée en comparant la concentration en 3-hydroxytétradécanoïque après et avant l'hydrolyse.
